# EUROPEAN PATENT APPLICATION

(11) **EP 3 287 134 A1**
(43) Date of publication of application: **28.02.2018**
(21) Application number: 16001872.7
(22) Date of filing: 26.08.2016
(51) Int. Cl.: A61K 35/14, A61K 35/62, A61P 19/00

(54) **COMBINATION THERAPY USING A BLOOD PREPARATION AND A HELMINTHIC PREPARATION**

(71) Applicant: Orthogen AG, 40212 Düsseldorf (DE)
(72) Inventor: WEHLING, Peter, 40597 Düsseldorf (DE); REINECKE, Julio, 50733 Köln (DE)
(74) Representative: König, Gregor Sebastian

(57) **Abstract**

The present invention relates to a blood preparation and a helminthic preparation and its use in therapy and non-therapeutic applications, wherein the blood preparation is preparable by a production method comprising the following steps: providing a blood sample collected from an organism and a vessel or container, contacting said blood sample with said vessel or a container, and incubating said blood sample in said vessel or container.

## Description

The present invention relates to the a blood preparation in combination with a helminthic preparation and its use in treatment of medical conditions as well as in improvement of other physical parameters.

Human health status includes various medical and non-medical parameters. Where such parameters are intended to be altered there are different options that range from change of lifestyle and habit to physical exercise and especially for disease states, administration of medication. For example orthopaedic conditions, such as a condition of the back are an ubiquitous challenge for patients, health care professionals and health insurance system. Treatment for lower back pain frequently includes lower back pain exercises. Further part of a treatment plan is a pain killer medication. Several over-the-counter and prescription medications are available to help reduce lower back pain. Often, a steroid medication is injected to deliver a powerful anti-inflammatory solution directly to the area that is the source of pain. Many medications reduce inflammation, which is often a cause of pain, while others work to inhibit the transmission of pain signals from reaching the brain.

Careful attention to pain management is a critical component of a patient's recovery, as acute or chronic low back pain can lead to depression, difficult sleeping, difficult exercising and stretching, all of which in turn can exacerbate and prolong a painful back condition. Each medication has multiple unique risks, side effects and drug (or food or supplement) interactions.

It is the problem of the present invention to provide novel means which allow for improving medical and non-medical conditions. Advantageously, such a means is fast and easy to produce and is cost effective. Also advantageously such means are well tolerable.

The problem is solved by a novel combination of a blood preparation with a helminthic preparation and its use. For conciseness the combination of the blood preparation and the helmintic preparation is denoted herein also as "the novel combination".

### Summary of the invention

The inventors have surprisingly found an effect on physical conditions when administering the novel combination. Without being bound to theory, the ingredients of the combination appear to interact to effectuate pathways that are not affected by each ingredient individually administered. The inventors have also observed an effect on mental conditions.

The blood preparation is preparable by a production method comprising the following steps: providing a blood sample collected from an organism and a vessel or container, contacting said blood sample with said vessel or a container, and incubating said blood sample in said vessel or container. The blood sample is preferably (1) a whole blood sample or (2) a whole blood sample from which cells, in particular erythrocytes have been depleted. The helminthic preparation comprises one or more of the following: a parasite, parasite extract, parasite egg, parasite egg extract, parasite larvae, parasite larvae extract, parasite cercariae, parasite cercariae extract and/or parasite secrete.

In a second aspect the present invention relates to non-therapeutic use of the novel combination for improving an organism's physiological and psychological (herein, "psychological" is interchangeably used to "mental") parameters measurable e.g. in the so-called SF36 assay. Within the context of the present invention, the problem is solved if one or more parameters in the SF-36 assay is improved. For example, parameters of SF-36 are determined at a first time point. Then the novel combination is administered to an organism, in particular a human-being, either singly or repeatedly (e.g. multiple administrations separated by intervals); the SF-36 parameters are re-determined. By comparing the re-determined values of the parameters to the initial values it is possible to determine whether there has been a change in these parameters as a result of the administration of the novel combination.

In a third aspect the present invention concerns a kit comprising the novel combination. That is, the blood preparation and the helminthic preparation are constituents of the kit.

### Detailed description of the invention

The present invention relates to a blood preparation and a helminthic preparation and its use in therapy and non-therapeutic applications. The invention is based on the finding that this novel combination has a significant effect on the SF36 score in human beings. In this respect it has been observed that some of the characteristics of the novel combination do not seem to coincide with some of the characteristics of the individual ingredient. For example the core characteristic of IL-1 Ra, as being a known component of the known blood preparation is an anti-inflammatory effect. However, treatment with the novel combination was observed not to be associated with a change in certain inflammatory parameters such as a reduction of the hs-CRP. This is even more surprising because not only IL-1Ra but also helminths are known to be anti-inflammatory when administered alone. The present combination appears to have no anti-inflammatory effect detectable by elevated hsCRP measurement which indicates that treatment is not or only to a neglectable extent based on a mechanism where inflammation plays a role. Rather, the effect of the novel combination is thought to be based on an balancing effect of the combination on the immune system.

Therefore, without being bound to theory it may be concluded that the novel combination modulates the immunological behaviour of the innate and/or the accuired immune system. More generally, it may be concluded that the novel combination has an effect on a condition caused by immune dysregulation. Immune dysregulation is a common hallmark of diseases with an autoimmune characteristics (including but not limited to RA, MS, Crohns, Fibromyalgia, Meniere, Glaucoma, Psoriasis), vaccination incompetence, various types of mental disorders including Autism Type Symptoms, Allergies, chronified degenerative diseases such as Osteoarthritis, Tendinitis, Osteoporosis, Periodontitis, chronic back pain, Parkinson, AD. These diseases or syndromes have very much varying causes and progressions as well as greatly differing complexities and consequences. Some are linked by a "co-segregation". Interstingly, in all of such pathologies an involvement of the immune system has been described.

One of the features of the immune system is an interplay between T cells and macrophages. In this regard, two major types of immune processes are commonly distinguished, Type 1 and Type 2. In the Type 1 processes, Type 1 T helper cells (Tₕ1 cells) and Type 1 macrophages (M1) are involved, and these processes play a major role in the cellular immune response and pathophysiology in inflammatory processes. The Type 2 processes involve Type 2 T helper cells (Tₕ2 cells) and Type 2 macrophages (M2) and play a role in anti-inflammatory and/or regenerative processes such as wound healing and tissue repair, besides their role in the humoral immune response. Typical cytokines associated with Type 1 processes are IFN-γ and IL-2. Type 1 immune response maximise the cellular killing ability. Where there is a (chronic) preponderance of Type 1 immune processes, damage to the organism may occur, e.g. when directed to autoantigens type 1 diabetes. The preponderance of Type 1 immune processes may e.g. be a preponderance of Type 1 over Type 2 immune processes. Such an imbalance in immune processes might be countered by promoting other immune processes (e.g. Type 2) or inhibiting Type 1 immune processes, facilitating the resolution of inflammation.

In many cases an overshooting "pro-inflammatory" reaction, namely Th1 or M1 polarization, is cause of some of many symptoms. In others, such as Asthma, an overshooting "anti-inflammatory" reaction such as Th2 or M2 polarization is involved in the symptomatics. This illustrates that while in many diseases "pro-inflammatory" in the sense of dysregulated Th1/M1 polarization needs to be alleviated, in others a dysregulated Th2/M2 polarization needs to be alleviated. Therefore a systemic and/or local rebalancing of the type1/type2 polarization is claimed to be a preferrential strategy.

INFg and other pro-inflammatory Th1/M1 cytokines are known to be involved in conditions of the CNS. There has been described an association of demyelination, schizophrenia, Parkinson disease, Alzheimer disease and bipolar disorders with microglia malfunction.

A M2a polarization of microglia reduces the severity of the so-called spreading depression (GLIA 2014;62:1176-1194. DOI: 10.1002/glia.22672 and Experimental Neurology 264 (2015) 43-54. http://dx.doi.org/10.1016/j.expneurol.2014.12.001). Furthermore, microRNA's altered expression was also reported in other human, non-cancer diseases including schizophrenia, neurodegenerative diseases like Parkinson's disease and Alzheimer disease, immune related disease, and cardiac disorders (Discoveries (Craiova). 2014 ; 2(4): e34-. doi:10.15190/d.2014.26). Many common immune-related diseases, including multiple sclerosis (MS), systemic lupus erythematosus (SLE), type I/II diabetes, and nonalcoholic fatty liver disease (NAFLD), have shown established correlations with cellular miRNAs Mechanistic studies revealed that miRNAs play critical roles in inflammation primarily by regulating the pathways associated with nuclear factor kappa beta (NF-κB), the central mediator of inflammatory response. For example, a systemic miRNA profiling in peripheral blood mononuclear cells from PD patients revealed miR-30b, miR-30c, and miR-26a to be associated with the susceptibility of the disease. Likewise, an analysis of miRNA and mRNA expression in brain cortex from AD and age-matched control subjects demonstrated strong correlations between the expression levels of miRNAs and predicted mRNA targets, implying functional relevance of microRNA-mediated regulations in AD pathogenesis (Genomics Proteomics Bioinformatics. 2012 Oct; 10(5): 246-253. doi: 10.1016/j.gpb.2012.07.005). miRNA expression profiling in human autoimmune diseases has inspired mechanistic studies of miRNA function in the pathogenesis of multiple sclerosis, rheumatoid arthritis, systemic lupus erythematosus, type 1 diabetes, and asthma (J Clin Invest. 2015;125(6):2242-2249. doi:10.1172/JCI78090.). The vesicles also have a sinister role in the propagation of toxic amyloid proteins in neurodegenerative conditions, including prion diseases and Alzheimer's and Parkinson's diseases, in inducing neuroinflammation by exchange of information between the neurons and glia, as well as in aiding tumor progression in the brain by subversion of normal cells (The Journal of Neuroscience, 2014, 34(46):15482-15489. DOI:10.1523/JNEUROSCI.3258-14.2014). Exosomes from IFNg stimulated Dendritic Cells (DC) may stimulate remyelinisation (Journal of Neuroimmunology 266 (2014) 12-23. http://dx.doi.org/10.1016/j.jneuroim.2013.10.014).

The secretom of M1 polarized macrophages stimulates CSPG synthesis in. CSPG plays a central role in the development of glial scars. The secretom of M2 polarized macrophages reduces inflammation induced damage (e.g. scar) (Open Journal of Regenerative Medicine, 2016, 5, 1-13. http://dx.doi.org/10.4236/ojrm.2016.51001).

Without being bound to theory it is thought that the blood preparation and the helminthic preparation, in particular the extracellular vesicles along with the cytokines and small RNA molecules enclosed in the vesicles have a positive influence on microglia and other macrophages of the CNS and the peripheral nervous system, either directly or indirectly by indirectly induced transmitter compounds, e.g. extracellular vesicles.

In one aspect the blood preparation is used in combination with the helminthic preparation in therapy. The term "therapy" as used herein denotes treatment of one or more conditions. Treatment also includes prevention and/or alleviation of one or more conditions. The term "condition" as used herein refers to disturbances in normal function or appearance and includes diseases, complications, disorders, syndromes, disabilities, stiffnesses, dysfunctions and/or symptoms thereof.

Preferably, the condition is selected from the group consisting of one or more of the following:
- pain;
- insomnia; and
- an orthopaedic condition.

The orthopaedic condition is preferably a musculoskeletal condition including pain, disability and/or stiffness of the musculoskeletal system.

The musculoskeletal condition is preferably a condition of the back, spine, knee, elbow, hip and/or ankle, in particular a condition of a joint, a bone or a disc thereof. The spine includes also the lumbar spine and/or cervical.

The joint condition is preferably arthritis. The bone condition is preferably osteoarthritis. In other embodiments the joint condition is a non-inflammatory joint condition and/or the bone condition is a non-inflammatory bone condition. In this context, it is particularly envisaged that the condition to be treated with the novel combination is a non-inflammatory condition at the time of treatment which without treatment would ultimately become inflammatory.

In a preferred embodiment, the condition is a chronic condition. In some embodiments it is preferred that the condition is a non-inflammatory condition and/or the condition is a result of a non-inflammatory condition.

Where embodiments of the present invention are described as "containing" or "comprising" certain subject matter, e.g. methods steps, constituents or other features, it is understood that preferred embodiments consist of said subject matter, except where the context dictates otherwise.

In another aspect of the invention the blood preparation is used in combination with the helminthic preparation for non-therapeutic use. In health care, health-related quality of life

(HRQoL) plays an increasing role in prevention of disease and is often measured by assays such as the SF36 assay. Preferably, the novel combination of blood preparation and helminthic preparation is used for improving quality of life, in particular health-related quality of life.

Health-related quality of life (HRQoL) is an assessment of how the individual's quality of life is affected over time by a condition. It focuses on the impact of health status on quality of life and includes physical, mental, emotional and social functioning aspects. HRQoL is commonly assessed using patient questionnaires. These are either disease specific or generic. The above mentioned SF 36 assay is a generic questionnaire and is the Short-Form Health Survey with 36 questions (SF-36) assessing quality of life, in particular physical and mental health-related quality of life.

The SF-36 consists of eight sub scores, which are the weighted sums of the questions in their section. The eight sections are vitality, physical functioning, bodily pain, general health perceptions, physical role functioning, emotional role functioning, social role functioning and mental health. Each sub score is scaled to 0-100 assuming equal weight of each question. The lower the sub score the worse a patient's subjective assessment. The higher the sub score the better a patient's subjective assessment. The results of the SF-36 questionnaire allow one for example to appreciate whether or not a treatment improves a patient's quality of life and/or health. In other words, the SF-36 may be used as a tool to assess success of a treatment or intervention.

According to the present invention, health and/or quality of life is determinable using SF-36 questionnaire and is therefore preferably determined using the SF-36 questionnaire. Within the context of the present invention, quality of life would be improved if one or more parameters in the SF-36 questionnaire improved, for example over time. For example, one or more, preferably all parameters of SF-36 are determined at a first time point. Then, at a later time point (i.e. after the novel combination was administered to an organism, in particular a human-being, either singly or repeatedly e.g. multiple administrations separated by intervals), the one or more, preferably all, SF-36 parameters are re-determined. By comparing the re-determined values of the parameters to the initial values it is possible to determine whether or not quality of life improved as a result of the administration of the novel combination. It is preferred that the one ore more parameters to be determined is one or more parameters of an organism's physical well-being and/or mental well-being.

In the context of the present invention it is conceived that an improvement of one or more SF-36 parameters by at least 5 %, preferably by at least 10 %, more preferably by at least 15 %, most preferably by at least 20 %, in particular by at least 25 % determined an improvement of health and/or quality of life. In some embodiment, the one or more SF-36 parameters are SF-36 sub scores. In preferred embodiments, the one or more SF-36 parameters are the SF-36 sum scores relating to physical and/or mental quality of life.

Alternatively or additionally, the health and/or quality of life can be determined using other systems including for example:
- Western Ontario and McMaster Universities Arthritis Index (WOMAC);
- Oswestry Index;
- Vernon and Mior Cervical Spine Questionnaire; and/or
- Visual Analogue Scale (VAS) for pain.

The WOMAC is a widely used, proprietary set of standardized questionnaires to evaluate conditions of patients with osteoarthritis of the knee and hip, including pain, stiffness, and physical functioning of the joints. According to the present invention, the WOMAC is preferably used to determine orthopaedic complaints.

The Oswestry Index is an index derived from the Oswestry Low Back Pain Questionnaire and, in the context of the present invention, is preferably applied to determine pain of the low back and disability for low back pain.

The Vernon and Mior Cervical Spine Questionnaire is preferably used to assess pain of the cervical spine and disability for cervical spine pain.

The VAS for pain is a measurement instrument that tries to measure a characteristic (i.e. pain) that ranges across a continuum of values and cannot easily be directly measured. The amount of pain that a patient feels ranges across a continuum from none to an extreme amount of pain. From the patient's perspective this spectrum appears continuous and its pain does not take discrete jumps, as a categorization of none, mild, moderate and severe would suggest. According to the invention, the VAS for pain is preferably used to determine pain.

Preferably the organism referred to herein is a human being. Also envisaged is an organism that is an animal, such as a dog, a cat, a horse or a camel. Said organism may or may not suffer from any of the herein described conditions.

The present invention further relates to a kit comprising the novel combination. That is, the blood preparation and the helminthic preparation are constituents of the kit. The skilled person will acknowledge that factors such as stability, administration routes and administration intervals (i.e. multiple administrations separated by intervals) of the blood preparation and the helminthic preparation, respectively, determine whether the preparations are contained in separate containers or whether they can be comprised in a single container. For example, if the blood preparation and the helminthic preparation are administered by the same route they may be combined prior to administration. In this case the kit may comprise the novel combination in a single container. If however the routes for administration differ, the kit preferably contains the blood preparation and the helminthic preparation in separate containers.

The kit may further contain instructions for use, for example, instructions for use in therapy and/or instructions for non-therapeutic use.

In the following preferred embodiments of the novel combination and its therapeutic and non-therapeutic use are described. These embodiments are to be understood to also describe preferred embodiments of the kit of the present invention.

The helminthic preparation of the novel combination preferably comprises one or more viable parasites. A viable parasite herein denotes a parasite having an intact metabolism and can be administered in the form of a developed parasite (e.g. worm) or a parasite egg, a parasite larva or a parasite cercaria being able to develop into a parasite having an intact metabolism.

In certain embodiments, the viable parasite is preferably a parasite that naturally colonizes the organism to which the novel preparation is intended to be given, preferably a human being. For example, the parasite may naturally reside in the human intestine (e.g. Trichinella spiralis; Fasciolopsis species, Echinostoma species, Heterophyses species), gut (e.g. *Ascaris lumbricoides, Enterobius vermicularis, Trichuris trichiura, Ancylostoma duodenalem, Necator americanus, Strongyloides stercoralis),* biliary system (e.g. Clonorchis sinensis, Opisthorchis viverrini, Opisthorchis felineus, Fasciola hepatica, Fasciola gigantica), venous system (e.g. Schistosoma species) and/or lung (lung flukes).

Accordingly, the parasite is preferably selected from the group of nematodes (in particular *Ascaris lumbricoides, Enterobius vermicularis, Trichuris trichiura, Ancylostoma duodenalem, Necator americanus, Strongyloides stercoralis* and *Trichinella spiralis*), platyhelminths (preferably trematodes and cestodes, in particular Fasciolopsis species, Echinostoma species, Heterophyses species, Clonorchis sinensis, Opisthorchis viverrini, Opisthorchis felineus, Fasciola hepatica, Fasciola gigantica, Schistosoma species, Diphyllobothrium species, Taenia saginata, Taenia solium and Hymenolepsis diminuta and Hymenolepsis nana), filarial and lung flukes.

More preferably, the parasite used in the helminthic preparation of the novel combination does not reproduce in the organism (in particular a human being) to which the novel preparation is intended to be given. The parasite is preferably one which does not naturally occur in said organism. It is intended that the parasite does not chronically infect/colonize the organism. Parasites that do not chronically infect an organism are usually those that do not naturally colonize that organism, in particular parasites that do not naturally infect human beings. Preferred parasites in this context are selected from the group consisting of Trichuris muris, Trichinella spiralis, Nippostrongylus brasiliensis, Heligmosomoides polygyrus and Hymenolepsis nana (naturally infect mice intestine); Trichuris suis and Ascaris suum (naturally infect pigs); Trichuris vulpis, Toxocara species, Toxascaris species, Gna-thostoma species and Ancylostoma species (naturally infect dogs or cats); Anisakis and Pseudoterranova (naturally infect marine mammals); and bird schistosomes such as S. douthitti, Trichobilharzia ocellata, T. stagnicolae, T. physellae, and Gigantobilharzia huronensis.

According to the invention the helminthic preparation of the novel combination does not need to comprise a viable parasite. In case it does not comprise a viable parasite, it may contain one or more non-viable components of helminthic parasites. In a preferred embodiment, the non-viable components are selected from the group consisting of a parasite extract, a parasite egg extract, a parasite larva extract, a parasite cercaria extract, a parasite secrete, a parasite egg secrete, a parasite larva secrete and a parasite cercaria secrete. An extract herein is defined as a substance or a mixture of substances, obtained from a helminthic parasite. An extract is typically extracted from the parasite. A secrete means a substance or a mixture of substances originating from a helminthic parasite. A secrete may be collected from the environment of the parasite. In both cases, the substance or mixture of substances comprises one or more active ingredients of the parasite resulting in the desired therapeutic or non-therapeutic effect (in particular in combination with the blood preparation). Typically, the extract or secrete is a complex mixture of substances. It will be understood that once identified the one or more active ingredients of the parasite can be isolated from any natural or non-natural source or synthetically produced and used in combination with the blood preparation as described herein. Most preferably, the non-viable components include exosomes. Exosomes can for example be prepared by collecting helminthic secretes from viable adult helminths (worms) held in vitro for several weeks.

A preferred embodiment in this context provides for combining the helminthic preparation and the blood preparation in a single formulation which is preferabyl parenterally administered. The single formulation preferably comprises or consist of exosomes stemming from the helminthic preparation and the blood preparation, respectively.

A specific and in some cases preferred non-viable component of helminthic parasites is a non-viable intact egg. It can be obtained by isolating viable eggs and killing eggs, for example by freezing in liquid nitrogen, thereby providing non-viable intact eggs. The procedure is described in WO 99/33470 on pages 17 to 19 with regard to providing non-viable, intact schistosome eggs, which procedure is herewith incorporated by reference.

Among the group of non-viable intact eggs particular preference is given to T. suis egg (TSO). The helminthic preparation comprising TSO is obtainable by a method comprising the steps of infecting an animal, such as a pig, with T. suis and isolating the eggs form the animal, in particular in the animal faeces, e.g. about 5 to 30 weeks, preferably about 5-11 weeks, more preferably about 7 to 9 weeks after inoculation. It is preferred that the isolation step comprises a washing procedure employing one ore more washing steps, e.g. employing sieves of decreasing mesh size (such as for example 1000, 500, 250, 100, 80, 70, 60, 50, and 20 µm mesh sizes), in order to wash and separate the eggs from e.g. undigested material in the faeces. It is preferred that the parasite eggs are contained in the sieved fraction and/or have a particle size of from 20-50 µm.

A T. suis egg preparation of the novel combination is also obtainable by a method comprising the step of recovering parasite eggs from worms isolated directly from the intestine of pigs. The isolated worms are preferably washed (optionally comprising one or more antibiotics in the washing solution) prior to the recovery of the eggs. It is preferred that the isolated worms are retained in vitro in growth medium, optionally supplemented with antibiotics, wherein the isolated worms lay their eggs. The eggs may be isolated from the growth medium by filtration on a sieve (e.g. 50 µm). The isolated/recovered eggs are preferably incubated in acidic medium (pH 0 to 7, preferably 0 to 5, more preferably 0 to 3, most preferably 0 to 2) at ambient temperature (preferably 15 to 30 °C) to allow for embryonation and inactivation of contaminating bacteria and viruses. To further prevent microbial growth an antibiotic can be optionally included. The embryonated eggs may then be added to a liquid carrier, in particular a pharmaceutically acceptable carrier to generate the helminthic preparation.

Most prefererably, the helminthic preparation comprises either viable adult helminths which are preferably incapable of reproducing within the organism to which the helminthic preparation is to be administered, in particular a human patient, or a non-viable component, in particular exosomes.

The blood preparation of the novel combination is preparable by a production method comprising the following steps: providing a blood sample collected from an organism and a vessel or container, contacting said blood sample with said vessel or a container, and incubating said blood sample in said vessel or container. The blood sample is preferably (1) a whole blood sample or (2) a whole blood sample from which cells, in particular erythrocytes have been depleted. In the following said production method is referred to as "the production method". It is preferred that said blood sample is a whole blood sample, according to the above alternative (1).

The above method allows for an easy production of a ready-to-use blood preparation without the need for special or complicated equipment and materials, without having to add any substances foreign to the body during production or such other substances which will have to separated again later in the production, comprising a minimum of steps and a few hours. By using exclusively a body's own substances, in this manner, an especially body-compatible agent is produced.

It is known that conditioned whole blood contains exosomes. Exosomes are small vesicles secreted from cells into their environment. Exosomes are for example contained in biological liquids such as serum, urine and synovial liquids. Most types of cells are able to secrete exosomes. The secretion occurs through release from the cell's plasma membrane. Depending on the cell type in which they are generated, exosomes contain inter alia a variable combination of proteins.

Preferably the blood preparation of the novel combination comprises exosomes. In certain preferred embodiments, the helmintic preparation comprises exosomes, too. The term "exosomes" preferably additionally comprises other extracellular vesicles. The extracellular vesicles preferably have an average diameter of 10 - 1000 nm.

Preferably exosomes contained in the blood preparation of the novel combination have been generated during the above-mentioned incubation of the blood sample (preferably whole blood sample) collected from the organism in a vessel or container. Exosomes contained in the helminthic preparation have preferably preparared by incubating viable adult helminths (worms) in vitro. It is envisaged that the blood sample and the adult viable helminths are incubated together in vitro, in particular the vessel or container, to generate exosomes.

The average diameter of the exosomes in an exosome-containing blood preparation and/or an exosome-containing helminthic preparation of the novel combination, as established by means of a transmission electron microscope, is preferably between 30 and 200 nm, in particular between 50 and 190 nm, between 70 and 180 nm, between 90 and 160 nm or between 100 and 150 nm. Exosomes of this size are the basis for an especially high efficacy, while larger vesicles represent essentially damaged exosomes and aggregates.

The production method preferably further comprises the step of concentrating or isolating the exosomes after the incubation in order to increase its efficacy further.

Such a concentrating or isolating step may lead to two or more blood preparations and/or two or more helminthic preparations of the novel combination prepared according to the production method. These two or more blood preparations correspond to the fractions obtained after performing such a concentrating or isolating step.

Concentrating or isolating the exosomes may for example be realised through centrifugation at 100,000 g, as such strong accelerations are especially suitable to concentrate or isolate exosomes. Such a centrifugation is preferably conducted for at least 30 min, especially for at least 60 min. The pellet formed by the centrifugation then contains the exosomes. Preferably the concentrated or isolated exosomes are then taken up in a liquid (preferably a buffered solution such as PBS). Optionally they are then filtrated, for example through a 0.2 µm filter.

The present invention also conceives a blood preparation of the novel combination that does not contain exosomes. However, a preferred blood preparation of the novel combination does comprise exosomes, and in particular it may consist of exosomes.

In case serum or plasma is contained in the blood preparation of the novel combination (which is preferred in certain cases), such serum or plasma preferably contains cytokines and/or growth factors.

Preferably the blood preparation of the novel combination does not comprise a corticosteroid, since this may impair efficacy, in particular due to inhibition of its senescence-rescuing effect and/or anti-apoptotic effect. Most preferably, the blood preparation of the novel combination comprises exosomes as defined above, and does not comprise a corticosteroid.

According to a preferred embodiment the production method comprises the following step: removing the cellular constituents of the blood sample (preferably whole blood sample) after the incubation and before administering the blood composition. Such a separation may be achieved by centrifugation, such as a short centrifugation at a low relative centrifugal force (e.g. about 10 minutes at 1000 g) or by filtration.

Incubation of the blood sample (preferably whole blood sample) is preferably carried out for a period of up to 72 hours, in particular a time period of 1 to 48 hours, 2 to 24 hours, 3 to 12 hours, 4 to 10 hours, 5 to 8 hours or 6 hours.

The incubation is preferably carried out at a temperature of 0°C to 45°C, in particular at temperatures of 10°C to 43°C, 20°C to 41°C, 30°C to 40°C, 35°C to 39°C, 36°C to 38°C or 37°C. These temperatures ensure best efficacy.

Suitable vessels or containers for carrying out the production method are for example hypodermic needles, tubes such as vacuum tubes, microtiter plates and transfusion bags. The vessel or container preferably comprises a surface for contacting the blood sample (preferably whole blood sample), which surface may comprise glass, plastic, corundum or quartz or a combination of these. A preferred plastic is selected from the group consisting of polystyrene, polycarbonate, polyethylene and polypropylene.

According to a preferred embodiment the vessel or container contains macroscopic particles, microscopic particles or nanoparticles and during incubation the blood sample is in contact with said particles. For the purposes of the present application, macroscopic particles are defined as particles that are visible when viewed with the naked eye, microparticles are defined as particles that are too small to be visible when viewed with the naked eye but are visible when viewed with a microscope, and nanoparticles are defined as particles that are too small to be visible when viewed with a microscope (and that are preferably larger than 1 nm). Such particles serve the purpose of enlarging the surface for contacting the blood sample and can have the shape of spheres, granulates, powder, gels or wool. Preferred materials are glass, plastic, corundum, quartz, gold and clay mineral (e.g. kaolin). Especially preferred are glass spheres. The surface of the particles can optionally be modified, for example by incubation with a caustic agent such as 50% v/v chromosulphuric acid with subsequent repeated rinsing.

Conditioned serum is also known as Orthokine®, which is used as the blood preparation of the novel combination in a preferred embodiment.

Preferably the blood preparation of the novel combination is for use in the treatment of the same organism from whom said blood sample (preferably whole blood sample) has been collected. In this case it is an organism that suffers from one or more of the above-mentioned conditions and/or whom quality of life is intended to be improved. Thus the blood preparation of the novel combination is preferably an autologous blood preparation and not an allogeneic one, especially for reasons of safety. When describing the present invention as the above-mentioned method of treating a condition and/or improving quality of life, this means that the organism and the patient are identical.

In order to achieve the maximum effect, it is preferred to avoid storage before using the blood preparation.

The efficacy of the blood preparation results from carrying out the herein described method steps, in particular the incubation step. In particular during the incubation step, efficacious components are induced in the blood sample, in particular due to the activity of cells therein. Therefore preferably said blood sample is a whole blood sample. However, the blood sample may also be a fraction of whole blood. For example erythrocytes, being cells that lack a nucleus and thus gene expression ability, may be absent from the blood sample. Therefore said blood sample is alternatively a whole blood sample from which erythrocytes and/or other cells have been depleted (preferably completely or substantially completely, but it is alternatively envisaged that only part of the erythrocytes have been depleted), for example a buffy coat or PRP (platelet-rich plasma). It is unnecessary to, and preferred not to, add any external stimulators or activators. Before the providing step, the production method preferably additionally includes the step of collecting said blood sample (preferably whole blood sample) from said organism. The blood preparation of the novel combination may be formulated as an injectable liquid or a solid (e.g. powder). The solid (e.g. powder) is preferably reconstituted with a pharmaceutically acceptable vehicle, e.g. water, prior to administration.

The helminthic preparation of the novel combination and/or the blood preparation of the novel combination are preferably formulated in a pharmaceutical formulation. The formulation in a pharmaceutical formulation is not limited to the therapeutic use of the novel combination but is also envisaged in the context of the non-therapeutic use. The pharmaceutical formulation preferably comprises a pharmaceutically acceptable carrier and may contain one or more pharmaceutically acceptable excipients, e.g. a preservative.

The helminthic preparation of the novel combination may be formulated as an oral dosage form (e.g. a tablet, a capsule, a liquid such as a solution or suspension), an injectable liquid or a solid (e.g. powder). The solid (e.g. powder) is preferably reconstituted with a pharmaceutically acceptable vehicle, e.g. water, prior to administration. The helminthic preparation of the novel combination may also be formulated as a rectal dosage form, in particular as a suppository. In a preferred embodiment, the blood preparation of the novel combination is used as provided by the aforementioned production method, preferably immediately after being produced.

The route of administration of the helminthic preparation of the novel combination depends on the specific parasite comprised in the helminthic preparation. In many cases the helminthic preparation of the novel combination is administered orally or parentally. Parenteral administration includes intravenous, intraarterial, intramuscular, intrathecal and subcutaneous administration, preferably intravenous, intramuscular and subcutaneous administration. In case the helminthic preparation of the novel combination comprises parasite eggs it is preferably administered orally. In case the helminthic preparation of the novel combination comprises parasite larvae or parasite cercaria it is preferably administered subcutaneously. In some cases, the helminthic preparation is administered rectally. In many cases it is preferred that the helminthic preparation is delivered to the intestine. In some cases the helminthic preparation of the novel combination is delivered to the systemic circulation or the gut, in rare cases to the lung. Preferably, the helminthioc preparation is administered orally. Most preferably, a helminthioc preparation comprising viable adult helminths that are incapable of reproducing within the patient is taken orally. Also preferred is the parenteral administration of non-viable helminthic components, in particular exosomes.

The blood preparation of the novel combination may be administered with local or systemic effect, in particular with systemic effect. In a particularly preferred embodiment, the blood preparation is delivered to the systemic circulation and the helminthic preparation is either delivered to the intestine or to the systemic circulation.

If the blood preparation of the novel combination and the helminthic preparation of the novel combination are administered by the same route they may be included in a single formulation which is administered using said same route. For example, the helminthic preparation is mixed with the blood preparation, preferably immediately after having produced the blood preparation, and administered, e.g. intravenously. In many cases, it is however preferred to administer the blood preparation and the helminthic preparation as separate formulations, in particular when different routes are envisaged. For example, the blood preparation may be often administered by the parenteral route and the helminthic preparation may be often administered by the oral route. Then, the two preparations are administered as separate formulations.

According to the present invention, each preparation of the novel combination is administered to an organism, preferably a mammal, in particular a human being, in an amount sufficient to achieve the desired therapeutic and non-therapeutic effect, respectively. In therapy, this amount is defined as therapeutically effective amount.

The helminthic preparation of the novel combination may be presented in unit dose or multidose form, each dose preferably comprising 3 to 300 parasites, more preferably 5 to 150 parasites, most preferably 10 to 100 parasites, in particular 20 to 50 parasites. Prefereably, the parasites are viable adult helminths that are incapable of reproducing within the patient.

In a preferred embodiment, the helminthic preparation is repeatedly administered (in intervals), for example every other week. Preferably, the helminthic preparation is administered in an interval of 0.5 weeks to 4 weeks. More preferably, the interval is 1 to 3 weeks. Most preferably, the helminthic preparation is administered biweekly. In a very preferred embodiment, the helminthic preparation is administered biweekly in a dose of 20 to 50 parasites each. Prefereably, the parasites are viable adult helminths that are incapable of reproducing within the patient.

The blood preparation is preferably repeatedly administered (in intervals), for example every 6 weeks. Preferably, the blood preparation is administered in an interval of 1 to 15 weeks. More preferably, the interval is 2 to 10 weeks. Most preferably, the blood preparation is administered every 3 to 9 weeks, in particular every 6 weeks.

Most preferably, both helminthic and blood preparation are repeatedly administered (in intervals), in particular in intervals of 1 to 3 weeks for the helminthic preparation and in intervals of 3 to 9 weeks for the blood preparation.

The helminthic preparation of the novel combination and/or the blood preparation of the novel combination is preferably administered for a prolonged time, e.g. for more than 1 month, preferably for 1 to 24 months, more preferably for 1 to 12 months, most preferably for 2 to 9 months, in particular for 3 to 6 months. During this time the helminthic preparation of the novel combination and the blood preparation of the novel combination are preferably administered in intervals, in particular in intervals of 1 to 3 weeks for the helminthic preparation and in intervals of 3 to 9 weeks for the blood preparation. The biweekly dose of the helminthic preparation preferably comprises 20 to 50 parasites.

### Examples

Example 1: SF-36 scores of patients treated with a blood preparation in combination with a helminthic preparation.

Patient (P2-P7) were treated for about 6 to 7 months. Each patient received a blood preparation every 6 weeks by intramuscular or subcutaneous injection. The blood preparation was prepared by obtaining a blood sample from each patient, treating each blood sample according to the production method described herein and delivering each treated blood sample (i.e. the blood preparation) back to the same patient from which the respective blood sample was obtained.

In addition, each patient received biweekly a helminthic preparations via oral route. The helmintic preparation comprised 30 viable adult helminths (worms) that are incapable of reproducing within a human patient.

Treatment was monitored using SF-36 questionnaires at time points F1 (before treatment), F2 (6 weeks after start of treatment) and F3 (about 6 to 7 months after start of treatment).

The results of the SF-36 questionnaires are shown in Tables 1 a, 1b and 1 c. In the tables the following abbreviations are used having the following meanings:
P2-P7: individual patients
   - Gesz: health state
   - KSK: physical health scores
   - PSK: mental health scores
   - KOFU: physical functioning
   - KÖRO: physical role functioning
   - SCHM: bodily pain
   - AGES: general health perceptions
   - VITA: vitality
   - SOFU: social role functioning
   - EMRO: emotional role functioning
   - PSYC: mental health

**Table 1a: SF questionnaires before treatment (F1).**

| | | Sum score | | Sub score | | | | | | | |
|---|---|---|---|---|---|---|---|---|---|---|---|
| | Gesz. | KSK | PSK | KÖFU | KÖRO | SCHM | AGES | VITA | SOFU | EMRO | PSYC |
| P2 | 3 | 52,86 | 55,28 | 85 | 100 | 84 | 87 | 70 | 100 | 100 | 80 |
| P3 | 4 | 44,82 | 36,70 | 60 | 75 | 51 | 62 | 35 | 63 | 67 | 56 |
| P4 | 3 | 36,59 | 56,38 | 65 | 50 | 41 | 65 | 60 | 88 | 100 | 76 |
| P5 | 4 | 42,00 | 33,07 | 60 | 100 | 22 | 52 | 10 | 63 | 33 | 68 |
| P6 | 5 | 34,39 | 27,73 | 60 | 0 | 22 | 25 | 55 | 38 | 0 | 48 |
| P7 | 2 | 48,38 | 58,44 | 100 | 100 | 62 | 62 | 75 | 100 | 100 | 88 |

**Table 1b: SF questionnaires 6 weeks after start of treatment (F2).**

| | | Sum score | | Sub score | | | | | | | |
|---|---|---|---|---|---|---|---|---|---|---|---|
| | Gesz. | KSK | PSK | KÖFU | KÖRO | SCHM | AGES | VITA | SOFU | EMRO | PSYC |
| P2 | 1 | 57,39 | 54,74 | 90 | 100 | 100 | 97 | 85 | 87,5 | 100 | 84 |
| P3 | 3 | 48,32 | 45,75 | 75 | 100 | 62 | 67 | 45 | 75 | 100 | 68 |
| P4 | 2 | 50,26 | 55,62 | 90 | 100 | 72 | 72 | 70 | 100 | 100 | 80 |
| P5 | 3 | 49,68 | 39,47 | 80 | 100 | 62 | 52 | 45 | 63 | 100 | 56 |
| P6 | 1 | 57,79 | 58,15 | 100 | 100 | 100 | 95 | 90 | 100 | 100 | 88 |
| P7 | 3 | 49,19 | 60,28 | 100 | 100 | 72 | 67 | 75 | 100 | 100 | 96 |

**Table 1c: SF questionnaires about 6 to 7 months after start of treatment (F3).**

| | | Sum score | | Sub score | | | | | | | |
|---|---|---|---|---|---|---|---|---|---|---|---|
| | Gesz. | KSK | PSK | KÖFU | KÖRO | SCHM | AGES | VITA | SOFU | EMRO | PSYC |
| P2 | 1 | 55,98 | 59,50 | 90 | 100 | 100 | 100 | 85 | 100 | 100 | 92 |
| P3 | 3 | 43,52 | 56,16 | 70 | 100 | 62 | 62 | 65 | 75 | 100 | 92 |
| P4 | 1 | 56,32 | 56,03 | 100 | 100 | 100 | 90 | 65 | 100 | 100 | 92 |
| P5 | 2 | 50,10 | 34,06 | 85 | 50 | 52 | 72 | 60 | 63 | 33 | 56 |
| P6 | 1 | 56,80 | 63,05 | 100 | 100 | 100 | 100 | 100 | 100 | 100 | 100 |
| P7 | 4 | 32,82 | 52,19 | 90 | 25 | 51 | 25 | 30 | 63 | 100 | 96 |

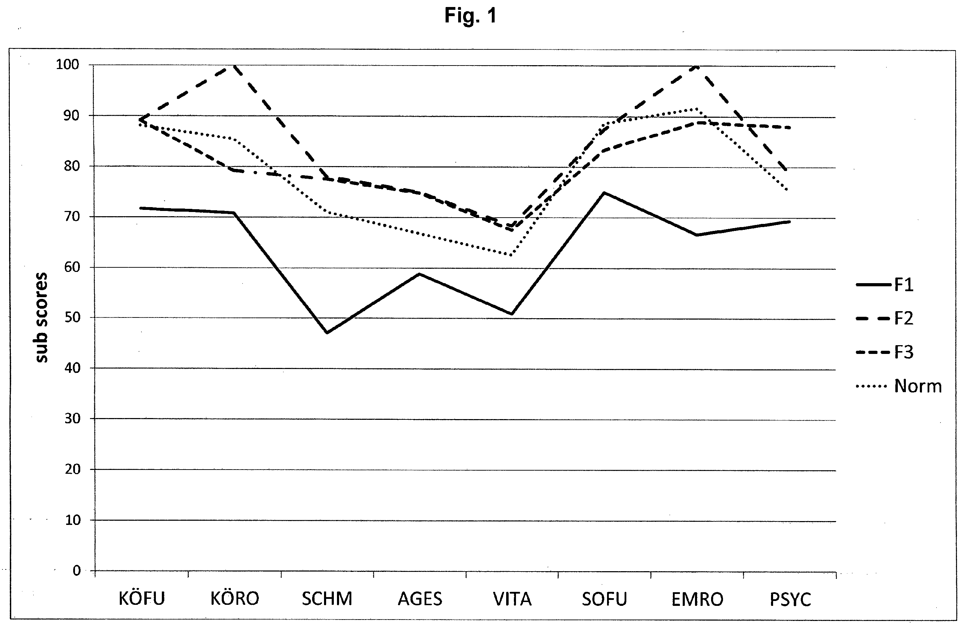

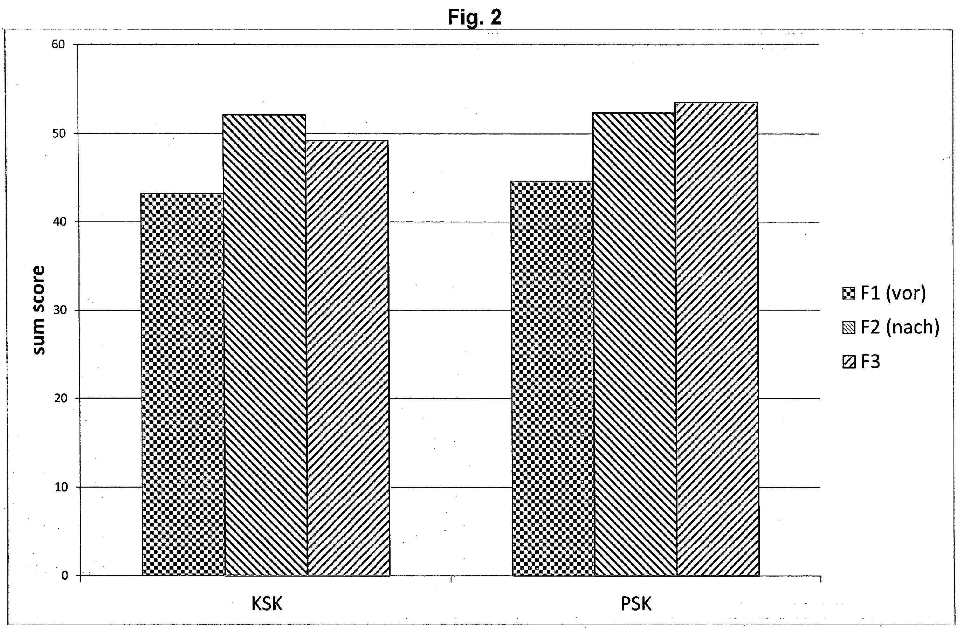

Figures 1 and 2 illustrate sub scores and sum scores over the course of treatment.

These results of the SF-36 questionnaires showed that treatment led to an improvement in SF-36 parameters relating to physical and mental well-being.

### Example 2: Analysis of patients treated with a blood preparation in combination with a helminthic preparation

From each patient of example 1 a blood count was taken at T1 (before treatment), T2 (about 6 weeks after treatment) and T3 (about 12 weeks after treatment). In addition, the patients' health was evaluated by SF-36 and the following measurement systems.

Orthopedic complaints were evaluated using the Western Ontario and McMaster Universities Arthritis Index (WOMAC). Disability for low back pain was evaluated using the Oswestry Index, an index derived from the Oswestry Low Back Pain Questionnaire. Pain of the cervical spine was assessed using the Vernon and Mior Cervical Spine Questionnaire. Pain was determined using a Visual Analogue Scale (VAS)

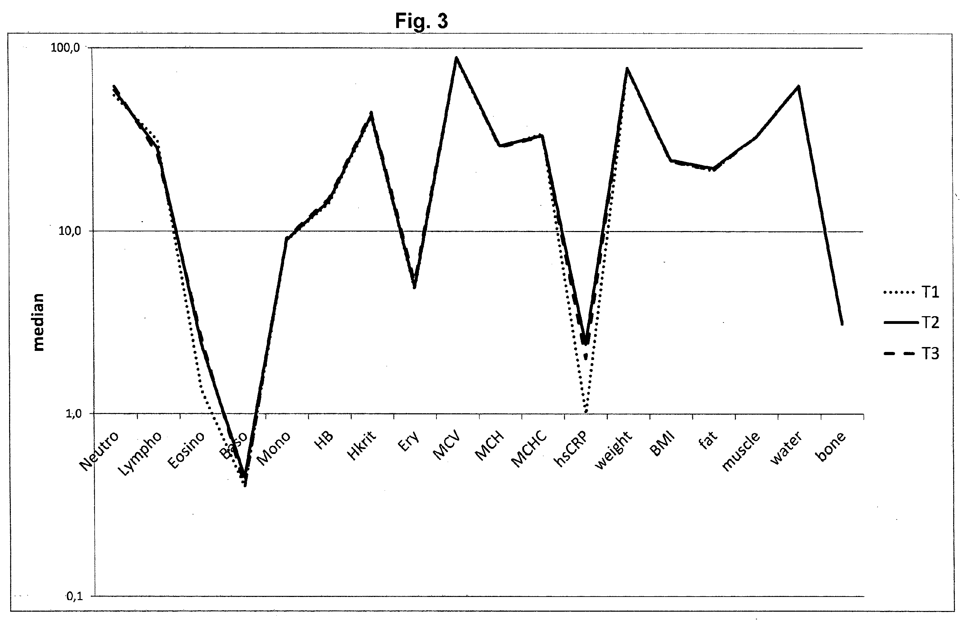

The results of patient's health status are shown in Table 2. The results of blood counts are depicted in Figure 3.

**Table 2: Health status of patients P2-P7 during course of treatment as determined by SF-36, WOMAC, Oswestry Index, Vernon and Mior Cervical Spine Questionnaire and VAS.**

| | | SF36 | | WOMAC | | | | | | |
|---|---|---|---|---|---|---|---|---|---|---|
| subject | time | KSK | PSK | pain | function | stiffness | total | Oswestry Index | Vernon Mior | VAS Ruhe |
| P2 | T1 | 52,86 | 55,28 | 3 | 5 | 0 | 8 | | | 9 |
| | T2 | 57,39 | 54,74 | 0 | 2 | 0 | 2 | | | 0 |
| | T3 | 55,98 | 59,50 | 1 | 3 | 0 | 4 | | | 0 |
| P3 | T1 | 44,82 | 36,70 | | | | | 20 | 22 | 28 |
| | T2 | 48,32 | 45,75 | | | | | 20 | 12 | 7 |
| | T3 | 43,52 | 56,16 | | | | | 24 | 4 | 43 |
| P4 | T1 | 36,59 | 56,38 | | | | | 22 | | 34 |
| | T2 | 50,26 | 55,62 | | | | | 12 | | 11 |
| | T3 | 56,32 | 56,03 | | | | | 0 | 0 | |
| P5 | T1 | 42,00 | 33,07 | 1 | 0 | 0 | 1 | | | 2 |
| | T2 | 49,68 | 39,47 | 3 | 9 | 0 | 12 | | | 3 |
| | T3 | 50,10 | 34,06 | 3 | 2 | 1 | 6 | | 1 | |
| P6 | T1 | 34,39 | 27,73 | 32 | 103 | 9 | 144 | 70 | 64 | 55 |
| | T2 | 57,79 | 58,15 | 0 | 0 | 0 | 0 | 0 | 0 | 0 |
| | T3 | 56,80 | 63,05 | 0 | 0 | 0 | 0 | 0 | 0 | 0 |
| P7 | T1 | 48,38 | 58,44 | | | | | | 0 | |
| | T2 | 49,19 | 60,28 | | | | | | 0 | |
| | T3 | 32,82 | 52,19 | | | | | 0 | 18 | 1 |

These results show that health status (e.g. Visual Analogue Scale (VAS)) improved considerably whereas no significant changes were observed in the patient's blood count (no correlation between VAS and blood count). The VAS correlated well with the SF-36. Patients P2, P4 and P6 showed strong improvements of the VAS relating to knee/hip complaints. This effect was temporarily observed in P3 (T2), too. Patients P3 and P6 showed considerable improvements in disability of the cervical spine.

Interestingly, treatment was not associated with a change in inflammatory parameters such as a reduction of the hs-CRP. This is surprising because both Orthokin® and helminths are anti-inflammatory when administered alone. The present combination appears to have no anti-inflammatory effect which indicates that treatment is not based on a mechanism where inflammatory plays a role.

### Figure Legends

- Fig. 1:: SF-36 sub scores for patients P2 to P7 as determined at time points F1, F2 and F3 using SF-36 patient questionnaires in comparison to normal. Sub scores relate to physical health scores (KSK), mental health scores (PSK); physical functioning (KÖFU); physical role functioning (KORO); bodily pain (SCHM); general health perceptions (AGES); vitality (VITA); social role functioning (SOFU); emotional role functioning (EMRO); mental health (PSYC).
- Fig. 2:: SF-36 sum scores for patients P2 to P7 as determined at time points F1, F2 and F3 using SF-36 patient questionnaires in comparison to normal. Main scores were determined from sub scores and relate to physical health scores (KSK) and mental health scores (PSK).
- Fig. 3: Analysis for patients P2 to P7 as determined at time points T1, T2 and F3. Levels of blood parameters are depicted as median values.

## Claims

1. A blood preparation for use in a combination therapy with a helminthic preparation, wherein the blood preparation is preparable by a production method comprising the following steps: providing a blood sample collected from an organism and a vessel or container, contacting said blood sample with said vessel or a container, and incubating said blood sample in said vessel or container, wherein said blood sample is (1) a whole blood sample or (2) a whole blood sample from which certain or all cells have been depleted.

2. A helminthic preparation for use in a combination therapy with a blood preparation, wherein the blood preparation is preparable by a production method comprising the following steps: providing a blood sample collected from an organism and a vessel or container, contacting said blood sample with said vessel or a container, and incubating said blood sample in said vessel or container, wherein said blood sample is (1) a whole blood sample or (2) a whole blood sample from which cells have been depleted.

3. The blood preparation or helminthic preparation for use according to claim 1 or 2, wherein the combination therapy comprises treatment of a condition selected from the group consisting of pain; insomnia; and an orthopaedic condition.

4. The blood preparation or helminthic preparation for use according to claim 3, wherein the orthopaedic conditions is a condition of the back, spine, knee, elbow, hip and/or ankle, in particular a condition of a joint, a bone or a disc thereof.

5. Non-therapeutic use of a blood preparation in combination with a helminthic preparation.

6. The preparation according to claims 1, 2 or 5 for use for improving a human's quality of life as determined by at least one parameter in the Short Form 36 Health Survey (SF-36).

7. The non-therapeutic use of claim 6, wherein the at least one parameter is a parameter of an organism's physical well-being and/or mental well-being.

8. The blood preparation or helminthic preparation for use according to claims 1 to 4, or the non-therapeutic use of claim 5 or 6, wherein the blood preparation and/or the helminthic preparation are repeatedly administered in intervals.

9. The preparation according to any one of claims 1 to 8, wherein the blood preparation is administered once per 1 to 15 weeks and/or the helminthic preparation is administered once per 3 days to once per 4 weeks.

10. The preparation according to any one of claims 1 to 9, wherein the blood preparation is administered parentally and/or the helminthic preparation is administered orally or parentally.

11. The preparation according to any one of claims 1 to 10, wherein the blood preparation is delivered to an organism's systemic circulation and/or the helminthic preparation is delivered to the organism's intestine or the organsim's systemic circulation.

12. The preparation according to any one of claims 1 to 11, wherein the blood preparation is administered to the same organism from which the blood sample has been collected.

13. The preparation according to any one of claims 1 to 12, wherein the helminthic preparation comprises a helminth, a helminth extract, helminth eggs, a helminth egg extract, helminth larvae, a helminth larvae extract, helminth cercariae, helminth cercariae extract and/or a helminth secrete.

14. The preparation according to any one of claims 1 to 13, wherein the helminthic preparation comprises 3 to 300 helminths.

15. The preparation according to any one of claims 1 to 14, wherein the helminthic preparation comprises helminths which do not naturally occur in a human and/or which are incapable of reproducing in the organsim and/or which does not chronically infect the organsim.

16. The preparation according to any one of claims 1 to 15, wherein said helminthic preparation and/or said blood preparation comprises or consists of exosomes..

17. The preparation according to any one of claims 1 to 16, wherein the average diameter of the exosomes as determined by transmission electron microscope is in the range between 30 and 200 nm.

18. The preparation according to any one of claims 1 to 17, wherein the blood preparation and the helminthic preparation are administered in a single formulation.

19. The preparation according to any one of claims 1 to 18, wherein the single formulation comprises or consists of exosomes from the blood preparation and the helminthic preparation.

20. A kit comprising a blood preparation and a helminthic preparation, wherein the blood preparation is preparable by a production method as defined in claim 1 and optionally, wherein the blood preparation and/or the helminthic preparation is as defined in any one of claims 12 to 19.

21. The kit according to claim 20 for use as a medicament.

22. The kit according to claim 21 for use in treating a condition as defined in claim 3 or 4.

23. The blood preparation for use according to claim 21 or 22, wherein the condition is non-inflammatory.
